# EUROPEAN PATENT APPLICATION

(11) **EP 4 494 665 A1**
(43) Date of publication of application: **22.01.2025**
(21) Application number: 24163769.3
(22) Date of filing: 15.03.2024
(51) Int. Cl.: A61L 26/00

(54) **HEMOSTATIC COMPOSITION AND HEMOSTASIS KIT INCLUDING SAME**

(30) Priority: 20.07.2023 KR 20230094425
(71) Applicant: Theracion Biomedical Co. Ltd., Seongnam-si 13202 (KR)
(72) Inventor: KIM, Eun Jin, Seongnam-si (KR); KIM, Byung Nam, Eumseong-gun (KR); JEON, Soo Hyeon, Gwangju-si (KR); JO, Hee Jung, Seongnam-si (KR)
(74) Representative: Ter Meer Steinmeister & Partner

(57) **Abstract**

The present disclosure relates to a hemostatic composition and a hemostasis kit including the same. More particularly, the present disclosure relates to a rapidly applicable hemostatic composition and a hemostasis kit including the same. The hemostatic composition according to the present disclosure includes a polysaccharide, a stabilizer, a binder, thrombin and an antibiotic, in which the polysaccharides is coated with the stabilizer, binder and thrombin, and the antibiotic is mixed with the coated polysaccharide. Since the hemostatic composition according to the present disclosure is provided in the form of a polysaccharide powder coated with a stabilizer, a binder, and thrombin, the hemostatic composition can be stored for a long period of time even at room temperature due to a large interaction area between the stabilizer and the thrombin, can be well fixed to the application site due to the binder, and has good hemostatic efficacy due to the high surface area of thrombin. In addition, the hemostatic composition can prevent infection at the bleeding site due to the presence of the antibiotic. In addition, the hemostatic composition may be mixed with a cationic solution that is separately stored, so that the hemostatic composition may be used as a hydrogel hemostatic agent if necessary. In addition, the powdered hemostatic composition can be housed in an endoscopic dispensing instrument to control bleeding during endoscopic procedures as needed, and thus can be used in a variety of bleeding situations.

## Description

### CROSS REFERENCE TO RELATED APPLICATION

The present application claims priority to Korean Patent Application No. 10-2023-0094425, filed July 20, 2023, the entire contents of which is incorporated herein for all purposes by this reference.

### BACKGROUND OF THE DISCLOSURE

### 1. Field of the Disclosure

The present disclosure relates to a hemostatic composition and a hemostatic kit including the same. More particularly, the present disclosure relates to a rapidly applicable hemostatic composition and a hemostatic kit including the same.

### 2. Description of the Related Art

In the medical field, the control of bleeding is essential and important during surgical procedures to minimize blood loss, reduce post-operative complications, and reduce operating time in the operating room. When blood, which is directly related to human life, leaks out of the body due to damage to blood vessels, various clotting factors act as enzymes, and calcium ions, thromboplastin, and other substances interact to form a fibrin network.

The blood corpuscles in the blood adhere to the fibrin network and aggregate to close the damaged area of the blood vessel, resulting in hemostasis. In this process, thrombin acts on fibrinogen to cleave fibrinogen peptides to produce fibrins, and a fibrine network is formed by electrostatic attraction between the fibrins. Fibrinogen has no hemostatic effect on its own, and its hemostatic effect is exhibited when it acts in conjunction with thrombin. In other words, thrombin plays an important role in the hemostatic response in the body.

However, hemostasis induced by the body's own hemostatic mechanism can provide sufficient hemostatic effect for mild bleeding such as capillary bleeding caused by minor wounds, but it is not effective for excessive bleeding that may occur during surgical operations or in emergency situations, and it is limited in stopping the bleeding sufficiently. Therefore, other effective hemostasis methods are required for the case of excessive bleeding. There are three main types of hemostasis methods: mechanical, cold, and chemical. However, the mechanical and cold hemostasis methods can cause secondary damage by affecting the tissue in and around the bleeding site. The chemical methods use biologic hemostatic substances such as thrombin and fibrinogen or drugs such as vasoconstrictors. The biologic hemostatic substances such as thrombin and fibrinogen are particularly suitable for hemostasis because they are present in the body and do not cause tissue damage.

The key function of a hemostatic agent is to quickly clot blood, thereby preventing excessive bleeding in various situations such as injuries and surgical procedures. Various drugs, polymeric materials, and proteins are currently used as hemostatic agents, among which thrombin preparations have excellent hemostatic effects.

Conventional thrombin preparations are liquid or powdered. Therefore, the thrombin preparations easily run off when applied to bleeding sites, whereby the hemostatic effect of thrombin cannot be fully exerted. To overcome this problem, WO1990/013320 discloses a sheet with thrombin immobilized on a bioresorbable material. However, the thrombin sheet was not put into commercial use because the thrombin sheet failed to show sufficient hemostatic effect and had difficulty in manufacturing and use thereof.

In addition, there are gel-type hemostatic agents made of a mixture of bovine thrombin and bovine gelatin or porcine collagen, but these hemostatic agents have the risk of causing infectious diseases such as bovine spongiform encephalopathy (BSE) because they contain polymers (gelatin, collagen, etc.) originating in animals. Particularly, collagen present in the body tissues slowly decomposes in the body, thereby causing inflammatory reactions in the case of organ transplantation or causing adhesions between tissues.

When oxidized regenerated cellulose (ORC), which is not derived from animals but is widely used as a topical dressing in various conventional surgical procedures, is mixed with thrombin, acidic residues present in cellulose denature the activity of thrombin, thereby rendering thrombin ineffective.

In addition, since thrombin, which is an enzyme isolated from blood, is unstable at room temperature, it is difficult to store thrombin for a long period of time. Thrombin is generally lyophilized and frozen for long-term storage, but there is a problem in that the activity of thrombin rapidly decreases in solution. Therefore, there is the inconvenience of having to take out the stored thrombin just before use.

Another known form of hemostatic agent is fibrin glue, which is a mixture of thrombin and other clotting factors. Fibrin glue, which is mainly composed of thrombin and fibrinogen, is a biological adhesive that utilizes the mechanism that thrombin converts fibrinogen to fibrin, and is used for the purpose of tissue adhesion, hemostasis, and wound closing. However, biological tissue adhesives containing fibrinogen must be frozen for storage and thus thawed for use, which is very inconvenient for use in urgent surgical procedures.

In order to solve the above-mentioned problems, there is a need for a hemostatic agent that does not cause denaturation of thrombin during mixing, prevents thrombin from flowing out when the hemostatic agent is applied to the body, contains a biocompatible and biodegradable polymer that can minimize infection factors that may be caused by the use of animal-derived polymers, has a long shelf-life at room temperature, and can sufficiently exert hemostatic effects.

The inventors made an effort to solve the above-mentioned problems and found that a hemostatic composition containing an antibiotic and a polysaccharide coated with a mixture of a stabilizer, a binder, and thrombin has a long shelf-life at room temperature, can be used to prepare a powdered or hydrogel hemostasis kit, and exhibits excellent hemostatic efficacy. On the basis of the finding, the inventors have completed the present disclosure.

### SUMMARY OF THE DISCLOSURE

An objective of the present disclosure is to provide a hemostatic composition that has a long shelf-life at room temperature, which can fix and apply thrombin promoting the formation of a fibrin network to a large bleeding area, and that contains an additive for minimizing the risk of infection. Another objective of the present disclosure is to provide a hemostasis kit including the same composition.

To achieve the above objectives, the present disclosure provides a hemostatic composition including (a) a polysaccharide coated with a mixture of a stabilizer, a binder, and thrombin; and (b) an antibiotic.

The present disclosure provides a powdered hemostasis kit including: (i) a hemostatic composition including (a) a polysaccharide coated with a mixture of a stabilizer, a binder, and thrombin and (b) an antibiotic; and (ii) a cationic substance.

The present disclosure provides an endoscopic powdered hemostasis kit including the powdered hemostasis kit and an endoscopic dispensing instrument.

The present disclosure provides a hydrogel hemostasis kit including: (i) a first container containing a hemostatic composition including (a) a polysaccharide coated with a mixture of a stabilizer, a binder, and thrombin, and (b) an antibiotic; (ii) a second container containing a cationic solution; and (iii) a connector connecting the first container to the second container.

Since the hemostatic composition according to the present disclosure is provided in the form of a polysaccharide powder coated with a stabilizer, a binder, and thrombin, the hemostatic composition can be stored for a long period of time even at room temperature due to a large interaction area between the stabilizer and the thrombin, can be well fixed to the application site due to the binder, and has good hemostatic efficacy due to the high surface area of thrombin. In addition, the hemostatic composition can prevent infection at the bleeding site due to the presence of the antibiotic. In addition, the hemostatic composition may be mixed with a cationic solution that is separately stored, so that the hemostatic composition may be used as a hydrogel hemostatic agent if necessary. In addition, the powdered hemostatic composition can be housed in an endoscopic dispensing instrument to control bleeding during endoscopic procedures as needed, and thus can be used in a variety of bleeding situations.

### BRIEF DESCRIPTION OF THE DRAWINGS

FIG. 1 is a photograph of a container for storing a powdered hemostatic composition according to the present disclosure;
FIG. 2 is a photograph of an endoscopic powdered hemostasis kit according to the present disclosure;
FIG. 3 is a photograph of a hydrogel hemostasis kit according to the present disclosure; and
FIG. 4 illustrates SEM images of a powdered hemostatic composition and a hydrogel hemostatic composition according to the present disclosure.

### DESCRIPTION OF THE PREFERRED EMBODIMENTS

The present disclosure provides a hemostatic composition including an antibiotic and a polysaccharide coated with a mixture of a stabilizer, a binder, and thrombin. The hemostatic composition can be stored for a long period of time at room temperature. In addition, (a) when the hemostatic composition is used in conjunction with a cationic substance and is provided in the form of powder, the hemostatic composition and cationic substance can contribute to hemostasis, can be easily applied to a large bleeding area, and can be fixed to the application site due to the binder. In addition, (b) when the hemostatic composition is used in conjunction with a cationic solution and provided in the form of hydrogel which can be topically applied to the exact target location. In this case, since the hemostatic composition does not flow down from the application site, the fixation effect of the hemostatic agent can be improved.

For the present disclosure, powder-type hemostatic compositions containing an antibiotic were prepared using various types of stabilizers, binders, and thrombin in specific amounts as a coating agent to coat polysaccharides, and (a) powdered hemostasis kits and (b) hydrogel hemostasis kits were prepared by mixing a cationic solution with the powdered hemostatic compositions. In addition, the hemostatic ability, toxicity, long-term storage effect, antibacterial activity, etc. of the hemostatic compositions and hemostasis kits were evaluated.

As a result, it was confirmed that the hemostasis kits according to the present disclosure has a long shelf-life even at room temperature because the hemostatic composition and the cationic solution are stored separately, has excellent hemostatic effects without toxicity, and has antibacterial effect of inhibiting adhesion and growth of bacteria.

Accordingly, in one aspect, the present disclosure relates to a hemostatic composition including: (a) a polysaccharide coated with a mixture of a stabilizer, a binder, and thrombin; and (b) an antibiotic.

In the present disclosure, the stabilizer stabilizes the activity of thrombin, thereby facilitating storage of the composition. The stabilizer may be, for example, serum albumin, mannitol, sorbitol, sucrose, trehalose dextran, glucose, sodium acetate, glycine, or the like. Among these, mannitol or sorbitol is preferred.

In the present disclosure, the binder is a component that comes into contact with the tissue of the bleeding site and cause the hemostatic composition to well adhere to the bleeding site. The binder may be, for example, Poly(vinyl pyrrolidone) (PVP), polyethylene glycol (PEG), polyethylene oxide (PEO), or the like. Among these, Poly(vinyl pyrrolidone) is preferred.

In the present disclosure, the polysaccharide may be, for example, starch, alginate, pullulan, carboxymethyl cellulose, dextrin, or the like. The polysaccharide is preferably starch, more preferably carboxymethyl starch, and even more preferably carboxymethyl starch having a molecular weight of 500,000 to 1,000,000 g/mol.

In the present disclosure, the antibiotic has antibacterial activity to prevent infection that may occur at the bleeding site. The antibiotic may be, for example, Gentamicin, Penicillin, Cephalosporin, Monobactam, Carbapenem, or the like. Among these, Gentamicin is preferred.

In the hemostatic composition of the present disclosure, the polysaccharide coated with the stabilizer, binder and thrombin mixture may include 1 to 45 wt% of the stabilizer, 2 to 10 wt% of the binder, 0.01 to 10 wt% of thrombin, and 35 to 96.99 wt% of the polysaccharide. The antibiotic may be included in an amount of 0.5 to 1.5 parts by weight per 100 parts by weight of the polysaccharide coated with the stabilizer, binder, and thrombin mixture.

When the content of the stabilizer is less than 1 wt%, there is a problem that thrombin is not stabilized whereby it is difficult to store the composition. When the content of the stabilizer exceeds 45 wt%, it is difficult to properly form a hydrogel when the composition is mixed with a cationic solution, and it is difficult to form a physical barrier at the bleeding site when the composition is used alone.

When the content of the binder is less than 2 wt%, the adhesion effect will be poor. When the content of the binder exceeds 10 wt%, the binder may interfere with the contact between the polysaccharide and the blood, thereby reducing the hemostatic effect.

When the content of thrombin is less than 0.01 wt%, the hemostatic effect is insignificant. When the content of thrombin exceeds 10 wt%, the manufacturing cost increases without improving the hemostatic effect, which is an economic problem.

When the polysaccharide content is less than 35 wt%, a hydrogel is not properly formed when the hemostatic composition is mixed with a cationic solution, and a physical barrier is not formed at the bleeding site when the hemostatic composition is used alone. When the polysaccharide content exceeds 96.99 wt%, it is difficult for the polysaccharide to mix with the stabilizer or thrombin.

When the antibiotic is contained in an amount of less than 0.5 parts by weight per 100 parts by weight of the polysaccharide coated with the stabilizer, binder, and thrombin mixture, the antibacterial activity is poor. When the amount of the antibiotic exceeds 1.5 parts by weight, there is no additional improvement in antibacterial effect, and the antibiotic is toxic.

In the present disclosure, the polysaccharide coated with the stabilizer, binder and thrombin mixture can be prepared by a method including the steps of (a) dissolving the stabilizer, binder and thrombin in distilled water to obtain a homogeneous solution; (b) immersing a polysaccharide in the homogeneous solution; (c) separating the polysaccharide from the solution; and (d) freeze-drying and grinding the separated polysaccharide.

The hemostatic composition including (a) the polysaccharide coated with the stabilizer, binder, and thrombin mixture and (b) the antibiotic, according to the present disclosure, is provided in the form of powder, whereby the hemostatic composition has a long shelf-life at room temperature.

In another aspect, the present disclosure relates to a powdered hemostasis kit including: (i) a hemostatic composition including (a) a polysaccharide coated with a mixture of a stabilizer, a binder, and thrombin and (b) an antibiotic; and (ii) a cationic substance.

In the present disclosure, the powdered hemostasis kit refers to a product including a container in which a hemostatic composition and a cationic substance are stored.

The hemostatic composition is the same as described above. The cationic substance is intended to assist the activity of thrombin and may be, for example, calcium chloride (CaCl₂), sodium chloride (NaCl), or a mixture thereof. Among these, calcium chloride (CaCl₂) is preferred.

In the present disclosure, the cationic substance is preferably included in an amount of 0.01 to 5 parts by weight per 100 parts by weight of the hemostatic composition. When the content of the cationic substance is less than 0.01 parts by weight, the cationic substance does not contribute sufficiently to the activity of thrombin. When the content of the cationic substance exceeds 5 parts by weight, the improvement in thrombin activity does not increase with increasing thrombin content, whereby it economically undesirable.

In the present disclosure, the hemostatic composition and the cationic substance can be stored in a container, and the material, shape, etc. of the container may be determined without any particular restriction, provided that the hemostatic composition and the cationic substance can be stored in the container. In the case of powdered forms, a bellows container is preferably used.

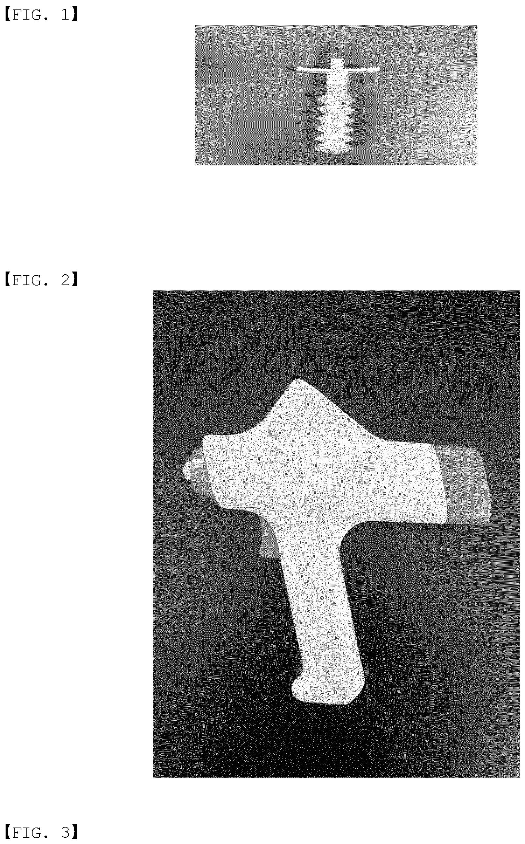

More specifically, as shown in FIG. 1, a bellows container includes a container for storing contents and a screwed cap that is fastened to a container end with an opening through which the contents escape. Preferably, the bellows container utilizes a structure and material that is compressible and recoverable in order to dispense a powder. The container may be, for example, a multipurpose medical powder dispensing device disclosed in Korean Patent No. 10-2391900.

The powdered hemostasis kit is ready to use in situations requiring the use of a hemostatic agent, allowing for immediate response in emergency situations.

Each of the prepared powdered hemostatic compositions was used in conjunction with an endoscopic dispensing instrument to control bleeding during endoscopic procedures. Each of the powdered hemostasis kits was used in conjunction with an endoscopic dispensing instrument, and the hemostatic capability thereof was evaluated during endoscopic procedures.

As a result, it was found that the powdered hemostasis kit of the present disclosure could be applied through an endoscopic dispensing instrument and had excellent hemostatic effects.

In a further aspect, the present disclosure relates to an endoscopic powdered hemostasis kit including the powdered hemostasis kit and an endoscopic dispensing instrument.

The endoscopic dispensing instrument according to the present disclosure can be used without any restrictions if it is possible to dispense a powdered hemostatic agent during surgical operations.

As illustrated in FIG. 2, the endoscopic dispensing instrument may include a container coupling portion to which the powdered hemostasis kit can be fastened, a tube coupling portion to which a tube passing through an endoscopic instrument is coupled, a motor to supply external air to the powdered hemostasis kit and the tube, a power supply to supply electric power to the motor, and a switch to regulate operation of the motor. The endoscope dispensing instrument may be, for example, a medical powder spraying apparatus that can easily regulate the flow rate of the sprayed powder and disclosed in Korean Patent No. 10-2443853.

In a further aspect, the present disclosure relates to a hydrogel hemostasis kit including: (i) a first container containing a hemostatic composition including (a) a polysaccharide coated with a mixture of a stabilizer, a binder, and thrombin, and (b) an antibiotic; (ii) a second container containing a cationic solution; and (iii) a connector connecting the first container to the second container.

The hemostatic composition is the same as described above. The cationic solution will be mixed with the hemostatic composition to form a hydrogel. The cationic solution may be, for example, calcium chloride (CaCl₂), sodium chloride (NaCl), or a mixture thereof. Among these, calcium chloride (CaCl₂) is preferred. The cationic solution may be prepared by adding a water-soluble solvent to 0.01 to 5 parts by weight of the cationic substance per 100 parts by weight of the hemostatic composition on a dry powder basis.

The first container of the hydrogel hemostasis kit of the present disclosure is a container for storing a hemostatic composition and the second container is a container for storing a cationic solution, and the material, shape, etc. of the containers can be used without any particular restrictions. However, it is preferable to use a typical syringe-shaped container.

More specifically, as illustrated in FIG. 3, the syringe-shaped container includes a container for storing contents, a screw cap that screws onto threads around an opening through which the contents escape from one end of the container, a push rod provided inside the container and configured to linearly reciprocate by physical force, and a piston coupled to one end of the push rod and located inside the container.

The connector is preferably in the form of a hollow tube that connects a portion of the first container to a portion of the second container so that the cationic solution in the second container can be transferred to the hemostatic composition contained in the first container, and the resulting solution composed of the cationic solution and the hemostatic composition can be transferred back to the second container.

The hemostatic composition in the form of powder contained in the first container is characterized in that it forms a hydrogel when mixed with a cationic solution, and the formed hydrogel has an average pore size of 100 pm to 1000 pm. When the average pore size of the hydrogel is smaller than 100 pm, the absorption rate of blood is reduced. When the average pore size exceeds 1000 µm, the hydrogel may not be able to maintain its shape after absorbing blood, resulting in a decrease in hemostatic effects.

The formation time of the hydrogel is in the range of from 5 seconds to 3 minutes, so that it is possible to prevent serious ligation and bleeding in emergency situations.

### [Example]

Hereinafter, the present disclosure will be described in more detail with reference to examples. These examples are presented only for illustrative purposes, and it will be apparent to those of ordinary skill in the art that the scope of the present invention is not to be construed as being limited by these examples.

### Examples 1 to 5: Preparation of hemostatic composition

A stabilizer (mannitol, sorbitol), binder (Poly(vinyl pyrrolidone)), and thrombin were added to 10 ml of distilled water according to a composition shown in Table 1 below, dissolved with an impeller, and homogenized to produce a homogeneous solution. Polysaccharide (carboxymethyl starch) with a molecular weight of 500,000 to 1,000,000 g/mol was immersed in the homogeneous solution. The immersed polysaccharide (carboxymethyl starch) was separated from the solution by passing the solution through a filter (45 um or smaller), and then the separated polysaccharide (carboxymethyl starch) was freeze-dried and ground to prepare a polysaccharide coated with a mixture of a stabilizer, binder, and thrombin. The coated polysaccharide was mixed with an antibiotic to prepare a hemostatic composition.

**[Table 1]**

| % by weight | polysaccharide coated with a mixture of a stabilizer, a binder, and thrombin | | | | | antibiotic |
|---|---|---|---|---|---|---|
| | carboxymethyl starch | mannitol | sorbitol | thrombin | poly(vinyl pyrrolidone) | gentamicin |
| Example 1 | 65 | 27 | - | 3 | 5 | 1.5 |
| Example 2 | 65 | - | 27 | 3 | 5 | 0.5 |
| Example 3 | 65 | 30 | - | 0.01 | 4.99 | 1.5 |
| Example 4 | 35 | 45 | - | 10 | 10 | 0.5 |
| Example 5 | 96 | 1 | - | 1 | 2 | 0.5 |

### Comparative Example 1 to 13: Preparation of hemostatic composition

A stabilizer (mannitol, sorbitol, collagen, gelatin), binder (poly(vinyl pyrrolidone)), and thrombin were added to 10 ml of distilled water to be a composition shown in Table 2 below, dissolved with an impeller, and homogenized to produce a homogeneous solution. Polysaccharide (carboxymethyl starch) was immersed in the homogeneous solution. The immersed polysaccharide (carboxymethyl starch) was separated from the solution by passing the solution through a filter (45 um or smaller), and then the separated polysaccharide (carboxymethyl starch) was freeze-dried and ground to prepare a polysaccharide coated with a mixture of a stabilizer, binder, and thrombin. The coated polysaccharide was mixed with an antibiotic to prepare a hemostatic composition.

**[Table 2]**

| % by weight | polysaccharide coated with a mixture of a stabilizer, a binder, and thrombin | | | | | | | antibioti c |
|---|---|---|---|---|---|---|---|---|
| | carboxymeth yl starch | collage n | gelati n | mannito 1 | sorbito 1 | thrombi n | poly (vinyl pyrrolidon e) | gentamici n |
| Comparati ve Example 1 | - | 65 | - | 27 | - | 3 | 5 | 1.5 |
| Comparati ve Example 2 | - | - | 65 | 15 | - | 15 | 5 | 1.5 |
| Comparati ve Example 3 | - | 65 | - | 32 | - | 3 | - | 0.5 |
| Comparati ve Example 4 | - | - | 65 | 32 | - | 3 | - | 0.5 |
| Comparati ve Example 5 | 65 | - | - | 30 | - | - | 5 | 1.5 |
| Comparati ve Example 6 | 65 | - | - | - | 30 | - | 5 | 0.5 |
| Comparati ve Example 7 | 65 | - | - | 32 | - | 3 | - | 2 |
| Comparati ve Example 8 | 65 | - | - | - | 32 | 3 | - | 1.5 |
| Comparati ve Example 9 | 70 | - | - | 29 | - | 0.01 | 0.99 | 0.5 |
| Comparati ve Example 10 | 30 | - | - | 65 | - | 3 | 2 | 1.5 |
| Comparati ve Example 11 | 55 | - | - | 4.99 | - | 0.01 | 40 | 1.5 |
| Comparati ve Example 12 | 97 | - | - | 0.95 | - | 0.005 | 2 | 0.5 |
| Comparati ve Example 13 | 86.95 | - | - | 0.05 | - | 3 | 10 | 0.4 |

### Preparation Examples 1 to 5: Preparation of powdered hemostasis kit

For the preparation of a powdered hemostasis kit, 0.5 to 1 g of one of the hemostatic compositions prepared in Examples 1 to 5 and 0.025 to 0.05 g of calcium chloride were placed in a bellows container.

### Preparation Examples 6 to 18: Preparation of powdered hemostasis kit

For the preparation of a powdered hemostasis kit, 0.5 to 1 g of each of the hemostatic compositions prepared in Comparative Examples 1 to 13 and 0.025 to 0.05 g of calcium chloride were placed in a bellows container.

### Preparation Examples 19 to 23: Preparation of powdered hemostasis kit

A powdered hemostasis kit was prepared by filling a bellows container with 0.025 to 0.05 g of calcium chloride and 0.5 to 1 g of one of the hemostatic compositions prepared in the same manner as in Examples 1 to 5, except that the polysaccharide was not coated with the stabilizer, binder, and thrombin.

### Preparation Examples 24 to 36: Preparation of powdered hemostasis kit

A powdered hemostasis kit was prepared by filling a bellows container with 0.025 to 0.05 g of calcium chloride and 0.5 to 1 g of one of the hemostatic compositions prepared in the same manner as in Comparative Examples 1 to 13, except that the polysaccharide was not coated with the stabilizer, binder, and thrombin.

### Preparation Examples 37 to 41: Preparation of hydrogel hemostasis kit

For the preparation of a hydrogel hemostatic kit, 0.5 to 1 g of the hemostatic composition prepared in one of Examples 1 to 5 was introduced into a syringe, and 2 to 5 ml of a 5% calcium chloride solution was introduced into the syringe.

### Preparation Examples 42 to 54: Preparation of hydrogel hemostasis kit

For the preparation of a hydrogel hemostatic kit, 0.5 to 1 g of the hemostatic composition prepared in one of Comparative Examples 1 to 13 was introduced into a syringe, and 2 to 5 ml of a 5% calcium chloride solution was introduced into the syringe.

### Preparation Examples 55 to 59: Preparation of hydrogel hemostasis kit

A hydrogel hemostasis kit was prepared by filling a syringe with 0.5 to 1 g of one of the hemostatic compositions prepared in the same manner as in Examples 1 to 5, except that the polysaccharide was not coated with the stabilizer, binder, and thrombin and then adding 2 to 5 ml of a 5% calcium chloride solution to the syringe.

### Preparation Examples 60 to 72: Preparation of hydrogel hemostasis kit

A hydrogel hemostasis kit was prepared by filling a syringe with 0.5 to 1 g of one of the hemostatic compositions prepared in the same manner as Comparative in Examples 1 to 13, except that the polysaccharide was not coated with the stabilizer, binder, and thrombin and then adding 2 to 5 ml of a 5% calcium chloride solution to the syringe.

### Experimental Example 1: Physical barrier formation test

A physical barrier formation test was performed on the hemostasis tic kits prepared in Examples 1-72 to confirm that the hemostatic compositions have the physical properties to exhibit hemostatic effect by determining physical barrier formation.

First, 1 g of the hemostasis kit prepared in one of Examples 1 to 36 was placed in a weighing dish, and 5 ml of distilled water was dropped in with a pipette. After 30 seconds, the hemostatic composition was visually checked for the formation of a physical barrier.

Next, a syringe filled with 1 g of the hemostatic composition prepared in one of Examples 37 to 72 and a syringe filled with 5 ml of 5% calcium chloride solution were mixed 20 times. The composition and solution were injection onto a weighing dish and visually checked to see if the hemostatic composition formed a physical barrier.

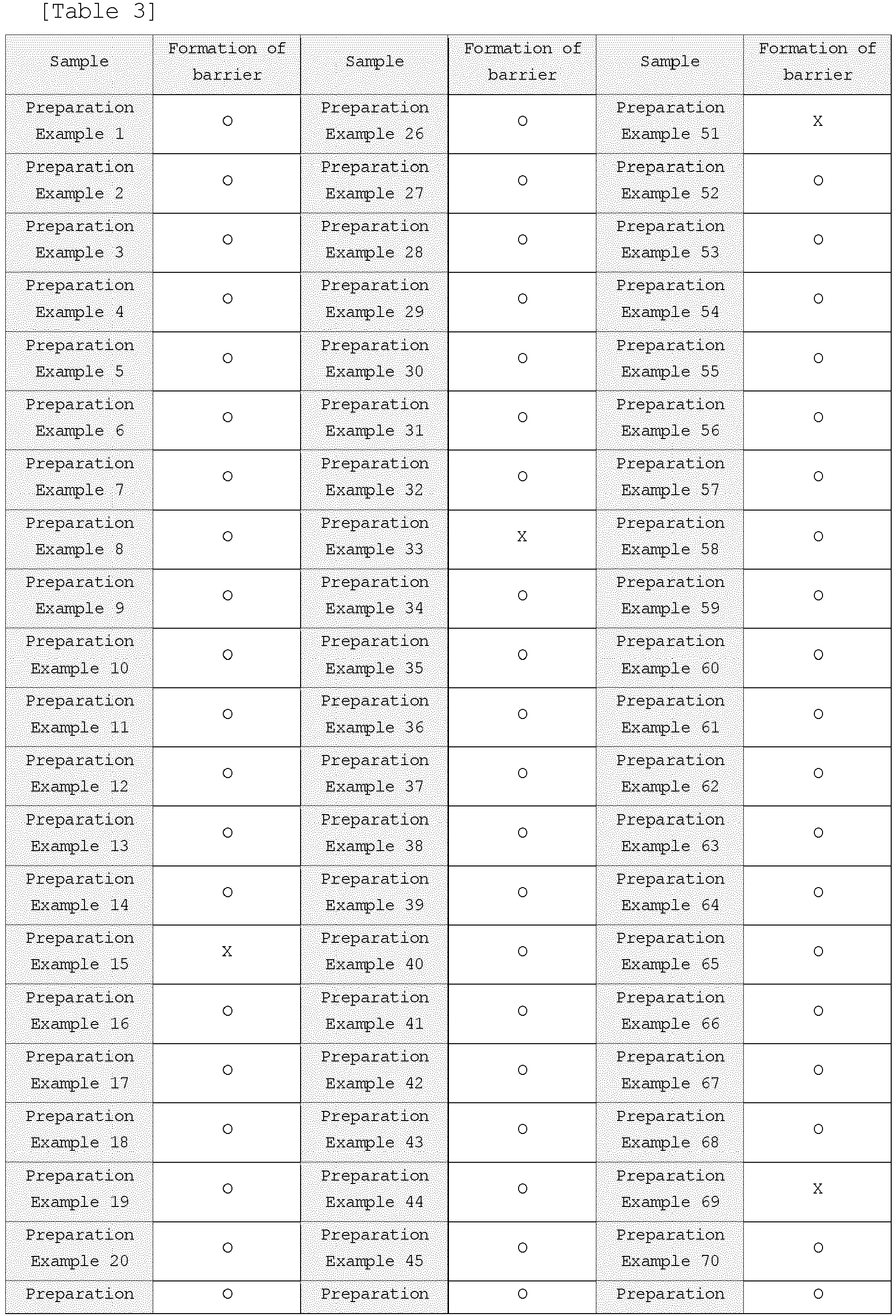

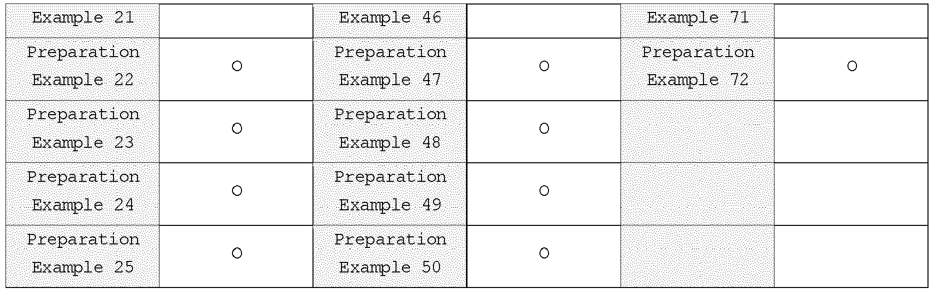

As shown in Table 3, the compositions prepared in Examples 15, 33, 51, and 69, which contained less carboxymethyl starch due to an overdose of the stabilizer, did not form a physical barrier, while the compositions prepared in the remaining examples formed a good physical barrier.

### Experimental Example 2: Adhesion evaluation test

An adhesion evaluation test was performed to determine whether the hemostatic compositions had the effect of being fixed to the bleeding site by checking the adhesion of the hemostasis kits prepared in the remaining preparation examples other than Preparation Examples 15, 33, 51 and 69 which failed to form a physical barrier in Experimental Example 1.

First, 1 g of the powdered hemostasis kit was placed on one end of an LDPE film, and 5 ml of distilled water was dropped with a pipette. After 5 minutes, the LDPE film was gradually tilted, and the time taken for the powdered hemostasis kit to move 10 cm from the initial position was recorded.

Next, a syringe filled with 1 g of the hydrogel hemostatic composition and a syringe filled with 5 ml of 5% calcium chloride solution were mixed 20 times, and 1 ml was placed on one end of the LDPE film. After 5 minutes, the LDPE film was gradually tilted, and the time taken for the powdered hemostasis kit to move 10 cm from the initial position was recorded.

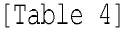

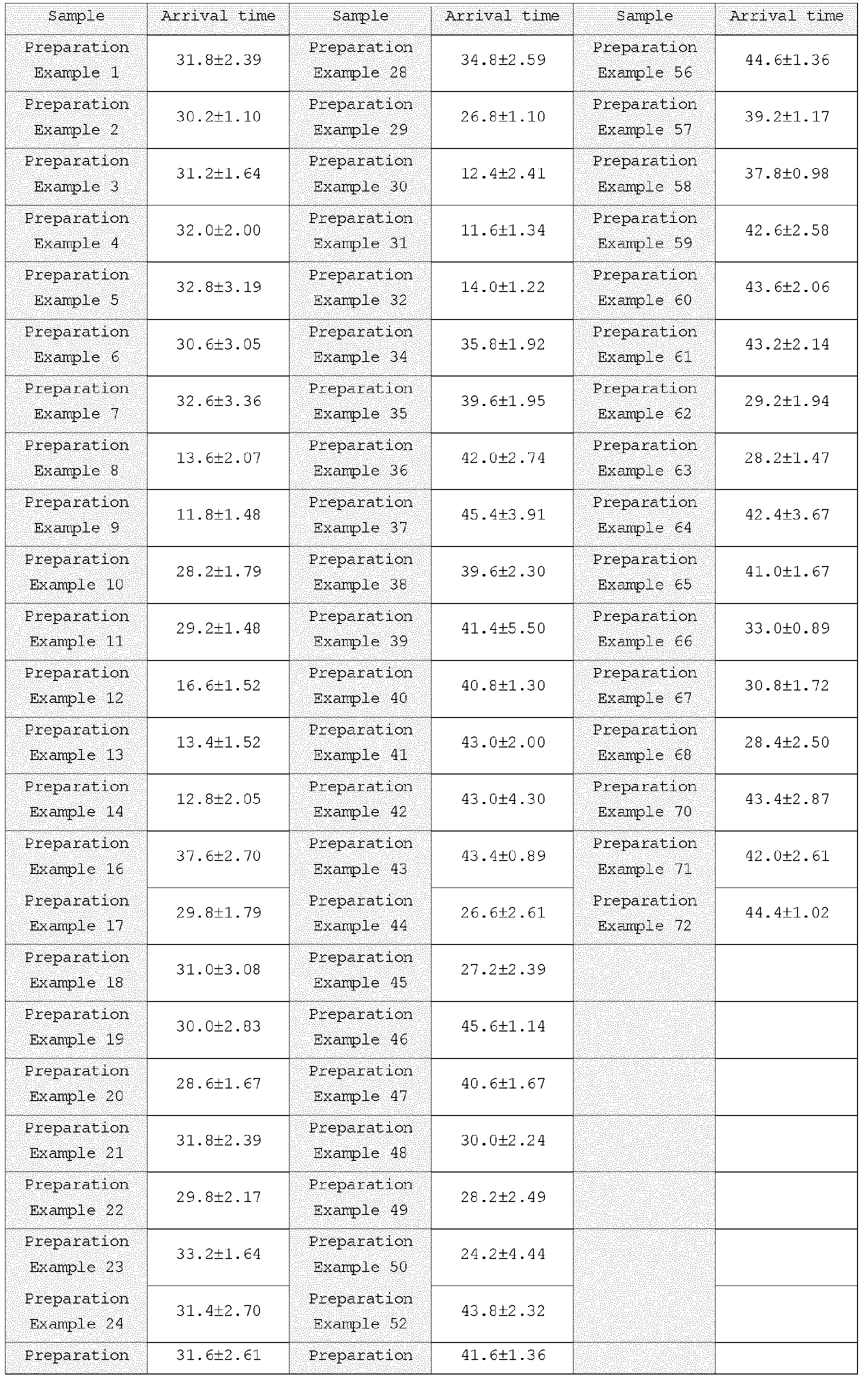

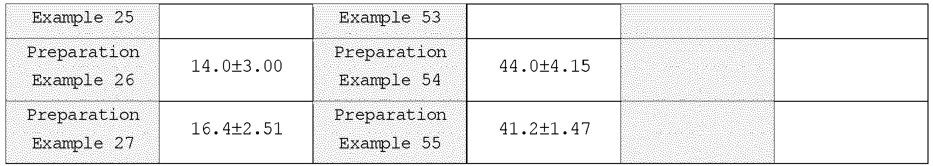

As shown in Table 4, Examples 8, 9, 12, 13, 26, 27, 30, 31, 44, 45, 48, 49, 62, 63, 66, and 67 without containing the binder were found to be less adhesive than those with the binder, and Examples 14, 32, 50, and 68 with less binder were also found to be less adhesive.

### Experimental Example 3: Compare in vitro hemostasis

To confirm the hemostatic ability of the hemostasis kits prepared in the preparation examples other than Preparation Examples 8, 9, 12 to 14, 26, 27, 30 to 32, 44, 45, 48 to 50, 62, 63, and 66 to 68, which exhibited less adhesion through Experimental Example 2, in an in vitro setting, a comparative clotting time test with dog whole blood was performed. Dog whole blood was added to a test tube, and 0.025 M aqueous calcium chloride solution was included in the blood to prevent interference of an anticoagulant. The sample was added at 37°C, and the tube was tilted at 5-second intervals to see if the blood clotted. When the blood did not clot, the blood was held stationary at 37°C for 5 seconds, and the clotting was rechecked. The process was repeated until the blood clotted, the time at which the blood clotted was recorded, and the results were plotted.

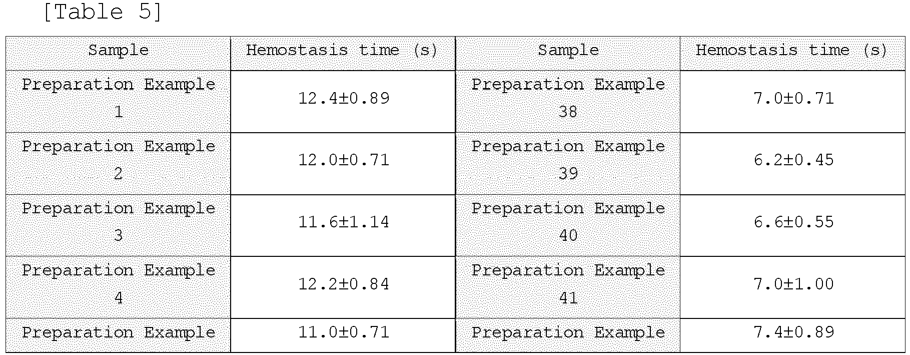

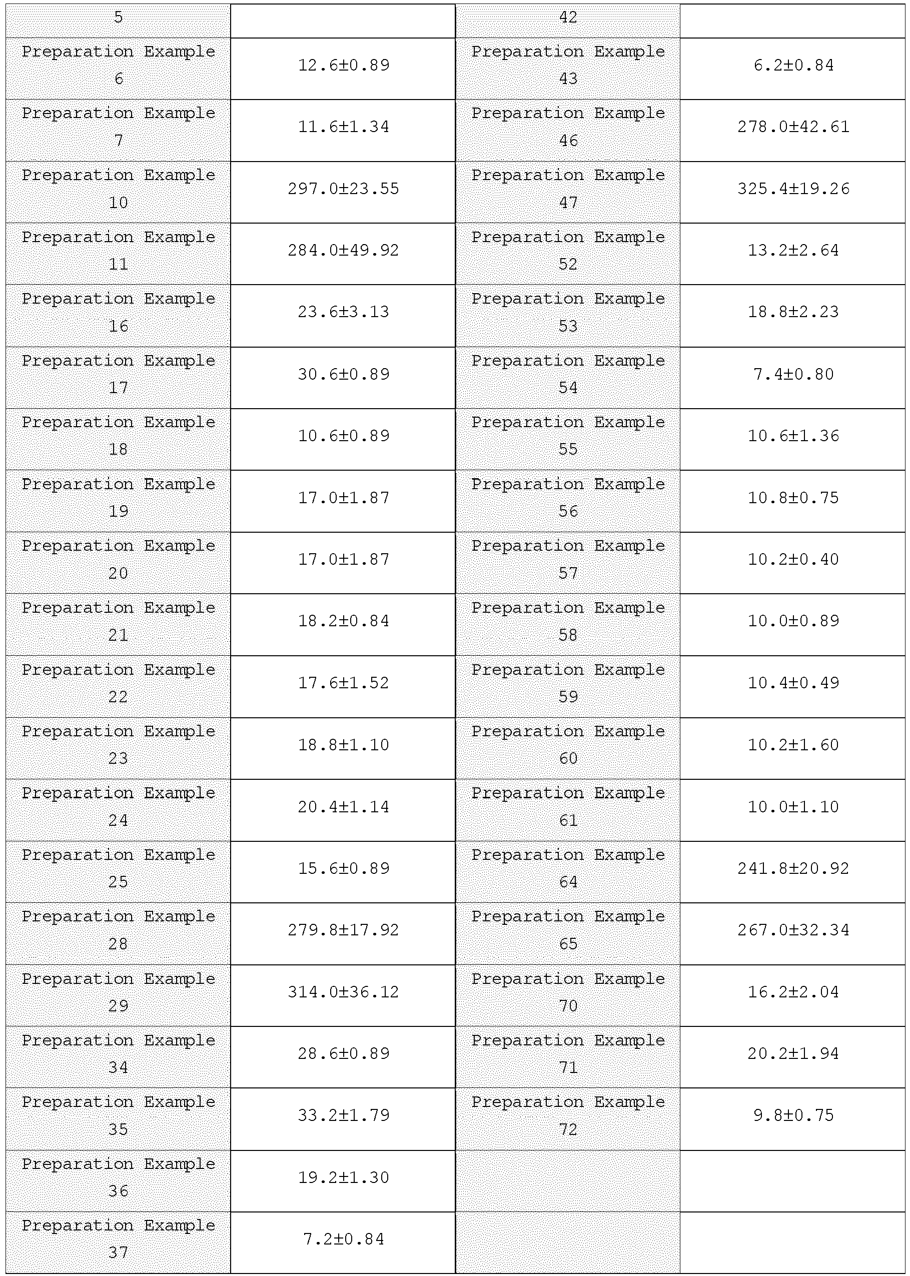

As shown in Table 5, Preparation Examples 10, 11, 28, 29, 46, 47, 64, and 65, which did not contain thrombin, were less hemostatic. In addition, Preparation Examples 16, 34, 52, and 70, which contained an excessive amount of binder, and Preparation Examples 17, 35, 53, and 71, which contained less thrombin, were relatively less hemostatic than the other preparation examples. When the binder was excessively contained, the binder interfered with contact between the thrombin and the blood, resulting in poor hemostasis. Preparation Examples 1 to 7, 18, 37 to 43, and 54 in which the polysaccharide was coated with the stabilizer, thrombin, and binder were found to have better hemostatic properties than Preparation Examples 19 to 25, 36, 55 to 61, and 72 in which the polysaccharide was not coated with the stabilizer, thrombin, and binder due to the coating. Preparation Examples 7, 25, 43, and 61 confirmed that the addition of excess thrombin had no effect on improving hemostasis.

### Experimental Example 4: Decomposition test

A decomposition test was performed on the hemostasis kits manufactured in the preparation examples other than Preparation Examples 10, 11, 16, 17, 19-25, 28, 29, 34 to 36, 46, 47, 52, 53, 55-61, 64, 65, and 70 to 72 which were found to be relatively low in hemostatic activity through Experimental Example 3, to ensure that there was no risk of inflammation due to the presence of non-degradable substances. First, the hemostatic composition was weighed. Each sample was then placed in a conical tube, and 10 ml of 1× SBF containing 0.05 mg/ml of amylase was added to the tube. The tube containing the sample was placed in a shaking incubator controlled to be at 37°C and 80 rpm and was held stationary for 24 to 120 hours. After 24, 48, and 72 hours, the samples were taken out, pressure filtered, dried, and weighed. The weight loss was calculated by introducing the weights of the samples measured before and after the decomposition test into the formula described below, and the weight loss was expressed as the degree of decomposition.

Calculation Equation Degree of decomposition (%) = (W0-Wt)/W0 * 100 (%)
WO: Initial weight (g), Wt: Later weight (g)

**[Table 6]**

| Sample | Weight reduction percentage (%) | | | |
|---|---|---|---|---|
| | 24H | 48H | 72H | 120H |
| Preparation Example 1 | 92.40 | 92.60 | 93.80 | 99.95 |
| Preparation Example 2 | 92.67 | 93.11 | 96.25 | 99.00 |
| Preparation Example 3 | 92.51 | 93.22 | 95.08 | 99.10 |
| Preparation Example 4 | 91.92 | 93.04 | 94.51 | 99.97 |
| Preparation Example 5 | 92.47 | 93.29 | 94.60 | 99.97 |
| Preparation Example 6 | 30.41 | 30.44 | 30.50 | 30.66 |
| Preparation Example 7 | 30.07 | 30.12 | 30.19 | 33.33 |
| Preparation Example 18 | 91.33 | 93.74 | 97.36 | 98.71 |
| Preparation Example 37 | 93.22 | 93.79 | 95.15 | 98.81 |
| Preparation Example 38 | 92.17 | 93.35 | 96.07 | 99.92 |
| Preparation Example 39 | 93.46 | 95.10 | 97.74 | 99.98 |
| Preparation Example 40 | 91.28 | 93.36 | 96.22 | 98.94 |
| Preparation Example 41 | 92.41 | 94.55 | 97.08 | 99.99 |
| Preparation Example 42 | 30.34 | 30.57 | 30.84 | 31.02 |
| Preparation Example 43 | 29.35 | 29.77 | 30.09 | 30.12 |
| Preparation Example 54 | 91.08 | 94.52 | 97.91 | 99.95 |

As shown in Table 6, Preparation Examples 1 to 5, 18, 37 to 41, and 54, which contained a polysaccharide, a stabilizer, a binder, thrombin, and an antibiotic, showed good decomposition, but Preparation Examples 6, 7, 42, and 43, which used collagen or gelatin instead of the polysaccharide, showed poor decomposition even after 120 hours.

### Experimental Example 5: Changes in thrombin activity with long-term storage

To determine whether the hemostatic compositions of Preparation Examples 1, 18, 37 and 54 according to the present disclosure maintain their thrombin activity during long-term storage at room temperature, the change in thrombin activity over an accelerated aging period was measured. Under accelerated aging conditions, each hemostatic composition was collected at a specific time (corresponding to real-time) and reacted with fibrinogen, and the time for fibrin formation was measured. The result was determined to be valid when the activity remained at least 80% of the initial value.

**[Table 7]**

| Storage period (month) | | 0 | 12 | 18 | 24 |
|---|---|---|---|---|---|
| Thrombin activity | Preparation Example 1 | 100% | 96.3% | 91.4% | 89.6% |
| | Preparation Example 18 | 100% | 80.7% | 73.4% | 67.8% |
| | Preparation Example 37 | 100% | 98.5% | 93.8% | 92.6% |
| | Preparation Example 54 | 100% | 84.2% | 80.1% | 78.9% |

As shown in Table 7, Preparation Examples 1 and 37 with adequate stabilizer maintained the activity of thrombin well over 24 months, while Preparation Examples 18 and 54 with less stabilizer showed a significant degradation of thrombin activity.

### Experimental Example 6: Changes in antibacterial power with antibiotic content

To determine the antibacterial power of the hemostatic compositions of Preparation Examples 1, 2, 12, 18, 37, 38, 48, and 54 according to the present disclosure, the degree of bacterial growth of *Staphylococcus aureus* was determined. An LB agar medium was prepared, 9 log CFU/ml of Staphylococcus aureus was streaked, and 1 g of the hemostatic composition was placed in the center of the medium. Staphylococcus aureus was grown at 37°C in an incubator for 24 hours and then visually checked for bacterial growth around the hemostatic composition. The composition was determined to be antibacterial when there was no bacterial growth around the composition.

**[Table 8]**

| Sample | | Inhibition of bacterial growth |
|---|---|---|
| Antibacterial power | Preparation Example 1 | ○ |
| | Preparation Example 2 | ○ |
| | Preparation Example 12 | ○ |
| | Preparation Example 18 | X |
| | Preparation Example 37 | ○ |
| | Preparation Example 38 | ○ |
| | Preparation Example 48 | ○ |
| | Preparation Example 54 | X |

As shown in Table 8, Preparation Examples 1, 2, 37, and 38, which contained adequate amounts of antibiotics, successfully inhibited bacterial growth, but Preparation Examples 18 and 54, which contained less antibiotics, did not inhibit bacterial growth well. Furthermore, it was observed that Preparation Examples 12 and 48, which contained an excessive amount of antibiotic, successfully inhibited bacterial growth, as did Preparation Examples 1 and 2.

### Experimental Example 7: Comparison of cytotoxicity in fibroblast

To confirm the safety of the powdered hemostatic compositions prepared in Preparation Examples 1, 2, 12 and 18 and the hydrogel hemostasis kits prepared in Preparation Examples 37, 38, 48 and 54, a comparative cytotoxicity test was performed in fibroblast. After culturing the fibroblast, a solution of the hemostatic composition eluted with a cell medium was added to a cell culture and incubated for 24 hours. The number of viable cells after the incubation was measured by MTT assay. The results are shown.

**[Table 9]**

| | Cell viability |
|---|---|
| Comparative group | 100% |
| Preparation Example 1 | 100% |
| Preparation Example 2 | 106% |
| Preparation Example 12 | 72% |
| Preparation Example 18 | 101% |
| Preparation Example 37 | 115% |
| Preparation Example 38 | 102% |
| Preparation Example 48 | 63% |
| Preparation Example 54 | 103% |

| | |
|---|---|
| * Comparative group Untreated group | |

As shown in Table 7, Preparation Examples 1, 2, 37 and 38 with adequate amounts of antibiotics were not cytotoxic with cell viability of 100% or more, whereas Preparation Examples 12 and 48 with excessive amounts of antibiotics were cytotoxic with decreased cell viability. It was also found that Preparation Examples 18 and 54, which contained less antibiotics, were not cytotoxic, as were Preparation Examples 1, 2, 37, and 38.

### Experimental Example 8: Comparison of hemostasis in rat liver injury model

To determine the hemostatic performance of the powdered hemostasis kit prepared in Preparation Example 1 and the hydrogel hemostasis kit prepared in Preparation Example 37, a comparative test of hemostatic performance was performed in a liver injury model. The liver was punctured to induce bleeding and then compressed with gauze for 30 seconds, followed by application of the hemostatic composition and compression for 15 seconds, and then checked for bleeding. When the bleeding continues, an additional 15 seconds of hemostatic compression was performed and repeated until hemostasis was complete. The time for successful hemostasis was measured, and the results are shown below.

**[Table 10]**

| | Hemostasis time (s) |
|---|---|
| Comparative group | 240.63 ± 26.98 s |
| Preparation Example 1 | 41.25 ± 9.92 s |
| Preparation Example 37 | 70 ± 29.05 s |

| | |
|---|---|
| * Comparative group: Treated with Gauze alone | |

### Experimental Example 9: Comparison of hemostatic performance in porcine gastrointestinal injury model

To determine the hemostatic performance of an endoscopic powdered hemostasis kit including the powdered hemostasis kit prepared in Preparation Example 1 and an endoscopic dispensing instrument, a comparative test of hemostatic performance was performed in a porcine gastrointestinal injury model. Bleeding classified as Forrest lb was induced by gastric mucosal excision using an endoscope, and the composition was applied using the endoscopic dispensing instrument. After 2 minutes of the application of the composition, it was checked whether the bleeding continued. When the bleeding continued, the composition was additionally applied, the injury was rechecked for bleeding after 1 minute. This process was repeated until hemostasis was achieved. The time for successful hemostasis was measured, and the results are shown below.

**[Table 11]**

| | Hemostasis time (s) |
|---|---|
| Comparative group | 434.20 ± 24.67 s |
| Preparation Example 1 | 140.20 ± 11.20 s |

| | |
|---|---|
| * Comparative group: Untreated with hemostatic agent | |

### Experimental Example 10: Confirmation of microstructure with scanning electron microscope (SEM)

To determine whether the hemostatic compositions according to the present disclosure form a hydrogel when mixed with a cationic solution, the microstructure was observed with SEM. The hemostatic composition (Example 1) not mixed with the cationic solution, and the hemostatic composition (Example 1) mixed with the cationic (calcium chloride) solution (Example 37) and freeze-dried were observed with SEM to obtain microstructure images.

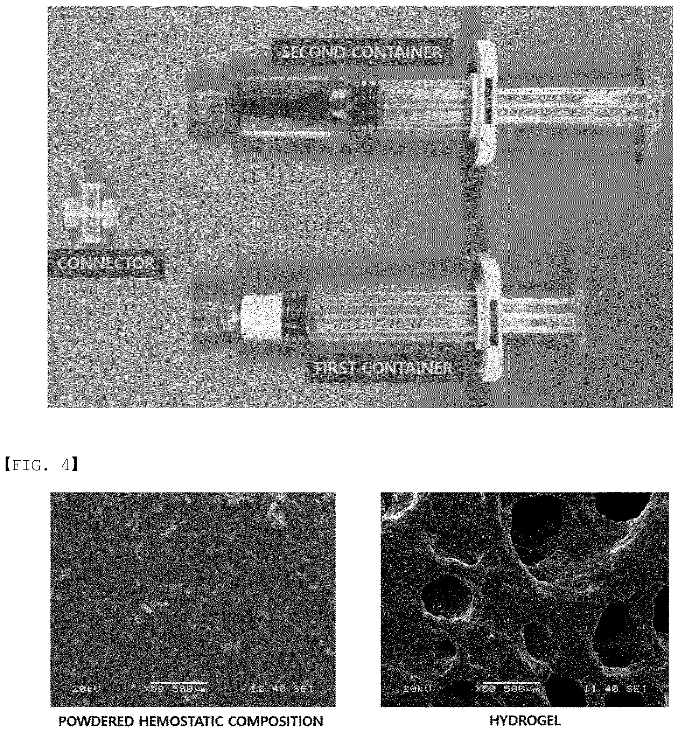

As a result, it was confirmed that the hemostatic composition in powder form was converted into a hydrogel by forming a porous network when the hemostatic composition was mixed with the cationic solution (FIG. 4).

Specific parts of the present invention have been described in detail, and those who ordinarily skilled in the art will appreciate that the specific parts described are only for illustrative purposes and the scope of the present invention is not limited by the specific parts described above. Accordingly, the substantial scope of the present invention will be defined by the appended claims and their equivalents.

## Claims

1. A hemostatic composition comprising: (a) a polysaccharide coated with a mixture of a stabilizer, a binder, and thrombin; and (b) an antibiotic.

2. The hemostatic composition of claim 1, wherein the stabilizer is at least one selected from the group consisting of serum albumin, mannitol, sorbitol, sucrose, trehalose dextran, glucose, sodium acetate, and glycine.

3. The pharmaceutical composition of claim 2, wherein the stabilizer is mannitol, sorbitol, or a mixture of mannitol and sorbitol.

4. The hemostatic composition of claim 1, wherein the binder is at least one selected from the group consisting of Poly(vinyl pyrrolidone) (PVP), polyethylene glycol (PEG), and polyethylene oxide (PEO) .

5. The hemostatic composition of claim 4, wherein the binder is Poly(vinyl pyrrolidone) (PVP).

6. The hemostatic composition of claim 1, wherein the polysaccharide is at least one selected from the group consisting of starch, alginate, pullulan, carboxymethyl cellulose, and dextrin.

7. The hemostatic composition of claim 6, wherein the polysaccharide is starch.

8. The hemostatic composition of claim 6, wherein the starch is carboxymethyl starch and has a molecular weight of 500,000 to 1,000,000 g/mol.

9. The hemostatic composition of claim 1, wherein the antibiotic is at least one selected from the group consisting of Gentamicin, Penicillin, Cephalosporin, Monobactam, and Carbapenem.

10. The hemostatic composition of claim 9, wherein the antibiotic is Gentamicin.

11. The hemostatic composition of claim 1, wherein the polysaccharide coated with the stabilizer, binder and thrombin mixture comprises 1 to 45 wt% of the stabilizer, 2 to 10 wt% of the binder, 0.01 to 10 wt% of thrombin, and 35 to 96.99 wt% of the polysaccharide, and the antibiotic is comprised in an amount of 0.5 to 1.5 parts by weight per 100 parts by weight of the polysaccharide coated with the stabilizer, binder and thrombin mixture.

12. The hemostatic composition of claim 1, wherein the polysaccharide coated with the stabilizer, binder and thrombin mixture is prepared by a method comprising:
(a) preparing a homogenous solution by dissolving the stabilizer, binder, and thrombin in distilled water;
(b) immersing the polysaccharide in the homogenous solution;
(c) separating the immersed polysaccharide from the solution; and
(d) freeze-drying and grinding the separated polysaccharide.

13. A powdered hemostasis kit comprising: (i) a hemostatic composition including (a) a polysaccharide coated with a mixture of a stabilizer, a binder, and thrombin and (b) an antibiotic; and (ii) a cationic substance.

14. The powdered hemostasis kit of claim 13, wherein the cationic substance is at least one selected from the group consisting of calcium chloride (CaCl₂) and sodium chloride (NaCl).

15. The powdered hemostasis kit of claim 14, wherein the cationic substance is comprised in an amount of 0.01 to 5 parts by weight per 100 parts by weight of the hemostatic composition.

16. An endoscopic powdered hemostasis kit comprising the powdered hemostasis kit of claim 13 and an endoscopic dispensing instrument.

17. A hydrogel hemostasis kit comprising:
(i) a first container containing a hemostatic composition comprising (a) a polysaccharide coated with a mixture of a stabilizer, a binder, and thrombin and (b) an antibiotic;
(ii) a second container containing a cationic solution; and
(iii) a connector connecting the first and second containers to each other.

18. The hydrogel hemostasis kit of claim 17, wherein the cationic solution is at least one selected from the group consisting of a calcium chloride (CaCl₂) solution and a sodium chloride (NaCl) solution.

19. The hydrogel hemostasis kit of claim 17, wherein the cationic solution is prepared by adding a water-soluble solvent to 0.01 to 5 parts by weight of the cationic substance per 100 parts by weight of the hemostatic composition on a dry powder basis.

20. The hydrogel hemostasis kit of claim 17, wherein when the hemostatic composition and the cationic solution are mixed, a hydrogel is formed, and the hydrogel has an average pore size of 100 pm to 1000 µm.
